# EUROPEAN PATENT SPECIFICATION

(11) **EP 4 534 140 B1**
(45) Date of publication and mention of the grant of the patent: **28.10.2026**
(21) Application number: 23720530.7
(22) Date of filing: 11.04.2023
(51) Int. Cl.: A61N 5/06

(54) **INTELLIGENT BIOLOGICAL VISION-CARE DEVICE FOR EYES**
INTELLIGENTE BIOLOGISCHE SEHPFLEGEVORRICHTUNG FÜR AUGEN
DISPOSITIF DE SOINS DE VISION BIOLOGIQUE INTELLIGENT POUR LES YEUX

(30) Priority: 24.05.2022 CN 202210575934
(43) Date of publication of application: 09.04.2025
(73) Proprietor: Shenzhen Chuying Shijie Health Technology Co., Ltd, Shenzhen, Guangdong 518000 (CN)
(72) Inventor: HUANG, Xiaoqiu, Shenzhen, Guangdong 518000 (CN)
(74) Representative: Chung, Hoi Kan
(86) International application number: PCT/CN2023/087682
(87) International publication number: WO 2023/226607

(56) References cited:
- CN-A- 112 137 849
- CN-A- 112 137 849
- CN-A- 114 870 275
- CN-U- 204 420 712
- CN-U- 204 798 346
- CN-U- 208 670 634
- CN-U- 208 818 984
- US-B1- 8 945 197

## Description

### TECHNICAL FIELD

The present disclosure belongs to the technical field of vision health, and in particular relates to an intelligent photo-biological treatment device for eyes.

### BACKGROUND

In recent years, due to work and lifestyle habits, our eyes have to work for an increasingly long time. In order to alleviate eye fatigue, eye massage machines emerged on the market. The existing eye massage machines perform mechanized or heated massages around the eyes to relax eye muscles and enhance their blood circulation. However, the existing eye massage machines can only temporarily alleviate eye fatigue, but cannot fundamentally improve vision health.

In a reference document D1 (CN208818984U), the utility model discloses a mobile phone display screen capable of improving display brightness. The touch screen comprises a backlight layer, a display layer, a touch layer and cover plate glass which are sequentially arranged from bottom to top, the backlight layer comprises an iron frame and a reflecting film, a light guide plate, a diffusion sheet, a lower brightness enhancement film, an upper brightness enhancement film and a brightness enhancement sheet which are sequentially installed in the iron frame from bottom to top, and the upper brightness enhancement film and the brightness enhancement sheet are bonded through black-black glue; the face, attached to the upper brightness enhancement film, of the brightness enhancement film is coated with a frosted coating. With the adoptionof the scheme, a frosted surface effect is added on the brightening sheet, so that the brightening sheet is prevented from being adsorbed with the upper brightening film, and the problems of Newton rings and rainbow lines are effectively solved; the frosted surface effect can also cover slight scratches, so that the production yield is improved; the frosted surface coating is added to separate the brightening sheet from the black adhesive of the backlight layer, so that the problem of edge delamination of the brightening film during disassembly can be solved; the backlight brightness can be improved by 5%-8%, and good market application value is achieved.

In a reference document D2 (CN112137849A), the invention discloses a bionic vision training instrument capable of achieving double light supplement for fundus posterior pole and fundus peripheral view. The biggest breakthrough point lies in that the bionic vision training instrument can achieve the visible light and infrared light double light supplement for the fundus posterior pole, especially for the fundus peripheral view. For example,when an infrared light supplementing emission source is arranged in the center of a window, omni-directional eye yoga exercise is carried out, when the eyeball just finds the peripheral light-emitting viewpoint, visible light supplements light for the fundus peripheral views, and infrared rays supplement infrared light for the fundus posterior pole; in the process that the eyeball tends to the light-emitting viewpoint, visible light is used for supplementing light in the peripheral view fields of the far position and the near position of a scratched part, and infrared rays are used for supplementing infrared light in the peripheral view fields of the near position and the far position of the scratched part; when the visual axis of the eyeball points to the peripheral light-emitting viewpoint, the visible light supplements light for the fundus posterior view, and the infrared light supplements light for the fundus peripheral view; and furthermore, blood circulation of peripheral visualfields of the near position and the far position of the scratched part is doubly and effectively improved, and the strength that the length of the eye axis of myopia hyperopia astigmatism tends to beforward is formed.

### SUMMARY

An objective of the present disclosure is to provide an intelligent photo-biological treatment device for eyes, to be used according to a described eye training method, in order to solve most if not all of the technical problems existing in the prior art.

The invention is set out in the appended set of claims.

The present disclosure has the following beneficial effects:

In the present disclosure, the first cavity and the second cavity are sequentially arranged on the side of each of the first cover and the second cover of the device body facing the human eye. The first cavity is provided therein with the light emission module, and the second cavity is provided therein with the dark training room in close contact with the human eye. The point light source of the light emission module is configured to emit 600-750 nm infrared light to stimulate the cone and rod cells in the retina of the human eye, causing the eye to secrete more dopamine. In this way, the blood vessel in the choroid of the human eye is dilated to strengthen the blood circulation in the retina and choroid, and move the thickening position of the wall of the blood vessel forward, thereby shortening the eye axis and improving uncorrected visual acuity (UCVA). The human eye observes the different colored light environments created by the photo-biological training room for a long time to relax ciliary muscles and restore their elasticity. The design improves the curvature adjustment ability of the eye lens, ensuring normal refractive adjustment of the eye. The music playback module is configured to play music, such as brainwave light music, to stimulate the brain's nervous system. The design can completely relax the brain nerves so as to relax the eye muscles of the vision system, further improving vision health.

### BRIEF DESCRIPTION OF THE DRAWINGS

FIG. 1 is a front view of an intelligent photo-biological treatment device for eyes according to an embodiment of the present disclosure;
FIG. 2 is a stereoscopic view of the intelligent photo-biological treatment device for eyes according to the embodiment of the present disclosure;
FIG. 3 is a structural view of an light emission module according to the embodiment of the present disclosure; and
FIG. 4 is a schematic diagram of a light emission control circuit according to the embodiment of the present disclosure.

Reference Numerals: 1. device body; 2. first cover; 3. second cover; 4. first cavity; 5. second cavity; 6. light emission module; 61. rubber-iron element; 62. reflective film; 63. light guide plate; 64. diffusive film; 65. lower brightness enhancement film; 66. upper brightness enhancement film; 67. point light source; and 68. black-black double-sided tape.

### DETAILED DESCRIPTION OF THE EMBODIMENTS

In order to make the objectives, technical solutions and advantages of the embodiments of the present disclosure clearer, the technical solutions in the embodiments of the present disclosure will be clearly and completely described below in conjunction with the drawings in the embodiments of the present disclosure. Obviously, the described embodiments are some, rather than all of the embodiments of the present disclosure. All other embodiments obtained by a person of ordinary skill in the art based on the embodiments of the present disclosure without creative efforts shall fall within the protection scope of the present disclosure.

### Embodiment

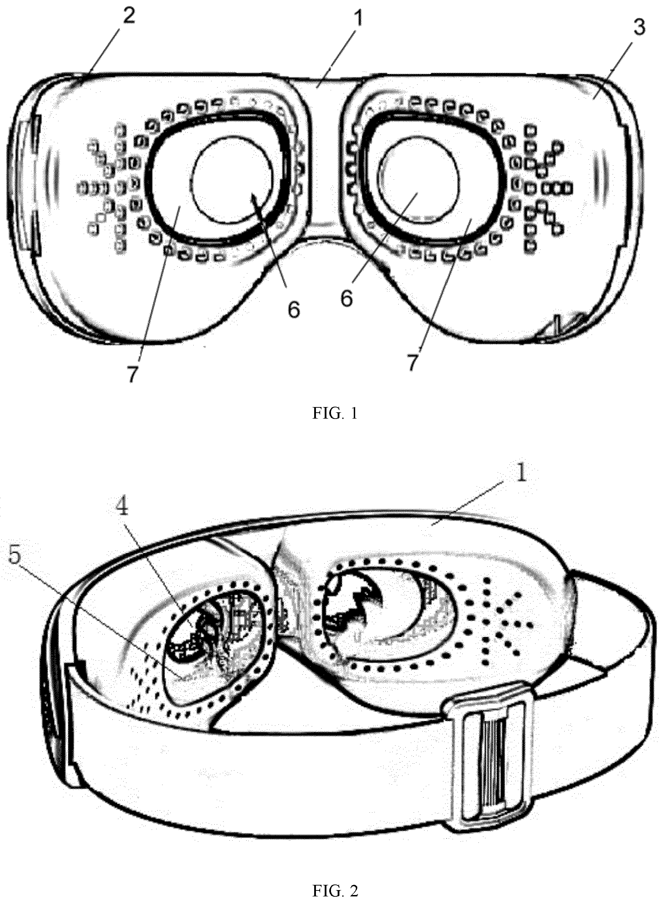

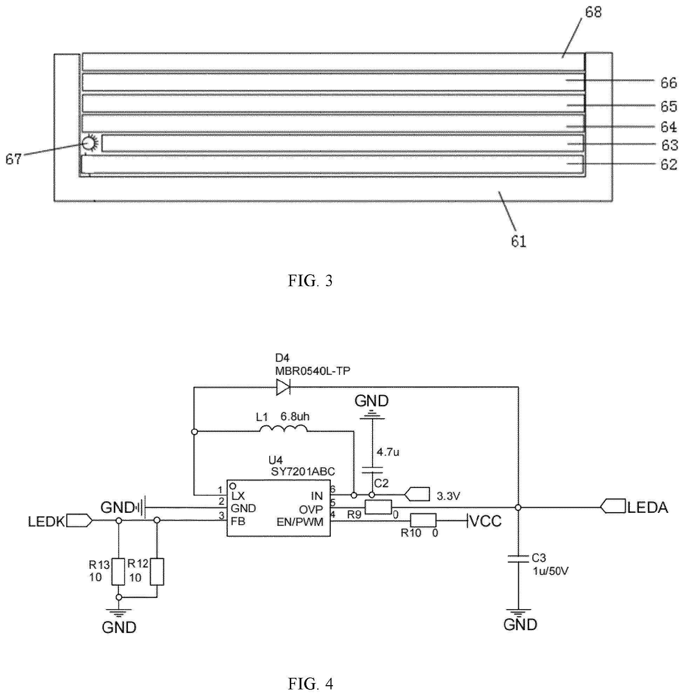

As shown in FIGS. 1 to 4, a first aspect of the present disclosure provides an intelligent photo-biological treatment device for eyes. The intelligent photo-biological treatment device for eyes includes a device body (1). The device body (1) includes a first cover (2) and a second cover (3) that are respectively adapted to left and right human eyes. A side of each of the first cover (2) and the second cover (3) facing the human eye is provided with a first cavity (4) and a second cavity (5) in order. The first cavity (4) is provided therein with a light emission module (6). Preferably, the light emission module (6) has a total thickness of no more than 0.95 mm. The second cavity (5) is provided therein with a photo-biological training room in close contact with the human eye.

The light emission module (6) includes a rubber-iron element (61). The rubber-iron element (61) includes an assembly groove. The assembly groove is provided therein with a reflective film (62), a light guide plate (63), a diffusive film (64), a lower brightness enhancement film (65), an upper brightness enhancement film (66), and a black-black double-sided tape (68) in order from bottom to top. The black-black double-sided tape (68) is bonded to an inner side of the first cover (2) or the second cover (3). A point light source (67) is located at one side of the light guide plate (63) and between the reflective film and the diffusive film (64). The point light source (67) is configured to emit 600-750 nm infrared light.

The device body (1) is provided therein with a controller (not shown in the figure). The controller is electrically connected with the point light source (67).

It should be noted that the assembly groove of the rubber-iron element (61) is configured to provide an assembly space for each component and plays a protective role. Preferably, the rubber-iron element (61) is integrally injection-molded. The reflective film is configured to reflect infrared light scattered from a back side of the light guide plate (63) back to the light guide plate (63), so as to increase the light brightness and uniformity. The light guide plate (63) is configured to convert the point light emitted by the point light source (67) into an area light source, and perform transmission and heat dissipation of the light to improve the uniformity of light transmission and scattering by the light guide plate (63). Preferably, in this embodiment, the light guide plate is made of polymethyl methacrylate (PMMA, also known as acrylic or organic glass) by laser engraving, and the laser-engraved light guide plate (63) has a V-CUT pattern. The light guide plate (63) has a uniformity of no less than 90%, while the existing light guide plate (63) has a uniformity of generally not less than 80%. There are differences in the flatness and uniformity of a dot matrix pattern printed on the existing light guide plate (63) using a traditional dot printing method, and the light transmission uniformity of the existing light guide plate can only achieve ≥ 80%. Therefore, the light guide plate (63) in this embodiment is superior to the traditional light guide plate (63), and is effectively suitable for stimulating and training biological cells in the retina of the human eye.

It should be noted that the diffusive film (64) is configured to further improve the uniformity of the area light source through a surface diffusion particle. The lower brightness enhancement film (65) and the upper brightness enhancement film (66) are respectively configured to focus and re-diffuse the infrared light source through a surface micro-pattern, so as to further improve the brightness and uniformity of the module. The point light source (67) has an emission angle of no less than 120°. The lower brightness enhancement film (65) and the upper brightness enhancement film (66) each are provided with a prism. The prism has an adjustable installation angle to generate focused light perpendicular to a direction of the human eye, so as to avoid overflow of the infrared light, and ultimately form infrared light perpendicular to the human eye at 90°. The design can achieve the goal of more concentrated and direct stimulation and activation of cone and rod cells in the retina of the human eye, which is beneficial for vision health. In contrast, a traditional white LED has an emission angle of not greater than 100°, such that the light emitted is scattered and cannot be focused. Therefore, this embodiment can be better used for stimulation and training of biological cells in the retina of the human eye.

Based on the disclosed content above, the first cavity (4) and the second cavity (5) are sequentially arranged on the side of each of the first cover (2) and the second cover (3) of the device body (1) facing the human eye. The first cavity (4) is provided therein with the light emission module (6), and the second cavity (5) is provided therein with the dark training room in close contact with the human eye. The point light source (67) of the light emission module (6) is configured to emit 600-750 nm infrared light to stimulate the cone and rod cells in the retina of the human eye, causing the eye to secrete more dopamine. In this way, the blood vessel in the choroid of the human eye is dilated to strengthen the blood circulation in the retina and choroid, and move the thickening position of the wall of the blood vessel forward, thereby shortening the eye axis and improving uncorrected visual acuity (UCVA). The human eye observes the different colored light environments created by the photo-biological training room for a long time to relax ciliary muscles and restore their elasticity. The design improves the curvature adjustment ability of the eye lens, ensuring normal refractive adjustment of the eye.

In a possible design, the device body (1) is further provided therein with a music playback module (not shown in the figure). The music playback module is electrically connected with the controller. The music playback module is configured to play music, such as brainwave light music, to stimulate the brain's nervous system. The design can completely relax the brain nerves so as to relax the eye muscles of the vision system, further improving vision health.

In a possible design, the point light source (67) includes a substrate layer. The substrate layer is provided thereon with a Bragg optical reflection layer. The Bragg optical reflection layer is provided thereon with an N-type aluminum gallium indium phosphide (N-AlGaInP) confinement layer with a thickness of 1,800-2,200 nm. The N-AlGaInP confinement layer is provided thereon with an I-AIGaInP active layer with a thickness of 0.1-0.3 µm. The I-AIGaInP active layer is provided thereon with a P-AIGaInP confinement layer with a thickness of 34,000-36,000 nm. The P-AlGaInP confinement layer is provided thereon with a p-type gallium phosphide (P-GaP) window layer with a thickness of 47,000-49,000 nm. The substrate layer includes a gallium arsenide (GaAs) substrate with a thickness of 220-320 nm. A surface of the GaAs substrate is provided with a silicon dioxide (SiO₂) protective layer with a thickness of 200-220 nm. A surface of the SiO₂ protective layer is provided with an indium tin oxide (ITO) enhancement layer with a thickness of 200-250 nm. The point light source (67) formed by connecting these layers can emit 600-750 nm infrared light.

In a possible design, in order to control the start and stop of the point light source, the controller includes a light emission control circuit. The light emission control circuit includes an SY7201ABC microprocessor chip U4. A pin LX of the microprocessor chip U4 is connected with one terminal of an inductor L1 and a positive terminal of the point light source. A negative terminal of the point light source is connected with a first capacitor C3 and then grounded. A ground pin GND of the microprocessor chip U4 is grounded. A pin FB of the microprocessor chip U4 is connected with one terminal of a first resistor R13, one terminal of a second resistor R12, and a positive pole of a power supply. The other terminal of the first resistor R13 and the other terminal of the second resistor are grounded. A pin IN of the microprocessor chip U4 is connected with the other terminal of the inductor L1, one terminal of a second capacitor C2, and a 3.3 V voltage. The other terminal of the second capacitor C2 is grounded. A pin OVP of the microprocessor chip U4 is connected in series with a third resistor R9 and then connected with one terminal of the first capacitor C3 and a negative pole of the power supply. Pins EN/PWM of the microprocessor chip U4 are connected in series with fourth resistor R10 and then connected with a power supply voltage VCC.

Preferably, in this embodiment, the parameters of each circuit component of the light emission control circuit are as follows: inductance of the inductor L1: 6.8 µH (micrhenry); capacitance of the first capacitor C3: 1 µ/50 V; resistance of the first resistor R13 and the second resistor R12: 10 ohms; capacitance of the second capacitor C2: 4.7 µ; resistance of the third resistor R9 and the fourth resistor R10: zero. An output constant current of the light emission control circuit is OUT=200 mV, and the inductance of the inductance L1 is not greater than 6.8 µH, otherwise a power supply integrated circuit (IC) may experience severe heating.

Here described is an illustrative example of an eye training method using the intelligent photo-biological treatment device for eyes in any one of the second to seventh possible designs in the first aspect, including the following steps.

The intelligent photo-biological treatment device for eyes is worn and positioned in front of human eyes. The controller controls the light emission module (6) to emit 600-750 nm infrared light perpendicular to the human eyes, and controls the infrared light to stimulate cone and rod cells in retinas of the human eyes for a first preset duration.

The human eyes face the photo-biological training room for an eye muscle relaxation training, and a voice prompt is provided when a second preset duration is reached.

In a possible design, the method further includes the following step.

The controller controls the music playback module to play music for a brain nerve stimulation training.

Based on the disclosed content above, in this embodiment, the point light source (67) of the light emission module (6) is configured to emit 600-750 nm infrared light to stimulate the cone and rod cells in the retina of the human eye, causing the eye to secrete more dopamine. In this way, the blood vessel in the choroid of the human eye is dilated to strengthen the blood circulation in the retina and choroid, and move the thickening position of the wall of the blood vessel forward, thereby shortening the eye axis and improving uncorrected visual acuity (UCVA). The human eye observes the different colored light environments created by the photo-biological training room for a long time to relax ciliary muscles and restore their elasticity. The design improves the curvature adjustment ability of the eye lens, ensuring normal refractive adjustment of the eye. The music playback module is configured to play music, such as brainwave light music, to stimulate the brain's nervous system. The design can completely relax the brain nerves so as to relax the eye muscles of the vision system, further improving vision health.

Finally, it should be noted that the above described are only preferred embodiments of the present disclosure and are not intended to limit the present disclosure. Any modifications, equivalent substitutions, improvements, etc. made within the scope of the present disclosure should be included within the protection scope of the present disclosure.

## Claims

1. An intelligent photo-biological treatment device for eyes, **characterized by** comprising a device body (1), wherein the device body (1) comprises a first cover (2) and a second cover (3) that are respectively adapted to left and right human eyes; a side of each of the first cover (2) and the second cover (3) facing the human eye is provided with a first cavity (4) and a second cavity (5) in order; the first cavity (4) is provided therein with a light emission module (6); and the second cavity (5) is provided therein with a photo-biological training room in close contact with the human eye;
the light emission module (6) comprises a rubber-iron element (61); the rubber-iron element (61) comprises an assembly groove; the assembly groove is provided therein with a reflective film (62), a light guide plate (63), a diffusive film (64), a lower brightness enhancement film (65), an upper brightness enhancement film (66), and a black-black double-sided tape (68) in order from bottom to top; the black-black double-sided tape (68) is bonded to an inner side of the first cover (2) or the second cover (3); a point light source (67) is located at one side of the light guide plate (63) and between the reflective film and the diffusive film (64); and the point light source (67) is configured to emit 600-750 nm infrared light; and
the device body (1) is provided therein with a controller; and the controller is electrically connected with the point light source (67);
wherein the point light source (67) comprises a substrate layer; the substrate layer is provided thereon with a Bragg optical reflection layer; the Bragg optical reflection layer is provided thereon with an N-type aluminum gallium indium phosphide (N-AlGaInP) confinement layer with a thickness of 1,800-2,200 nm; the N-AlGaInP confinement layer is provided thereon with an I-AIGaInP active layer with a thickness of 0.1-0.3 µm; the I-AIGaInP active layer is provided thereon with a P-AIGaInP confinement layer with a thickness of 34,000-36,000 nm; and the P-AlGaInP confinement layer is provided thereon with a p-type gallium phosphide (P-GaP) window layer with a thickness of 47,000-49,000 nm; and
the substrate layer comprises a gallium arsenide (GaAs) substrate with a thickness of 220-320 nm; a surface of the GaAs substrate is provided with a silicon dioxide (SiO₂) protective layer with a thickness of 200-220 nm; and a surface of the SiO₂ protective layer is provided with an indium tin oxide (ITO) enhancement layer with a thickness of 200-250 nm;
wherein the light guide plate (63) is made of polymethyl methacrylate (PMMA) by laser engraving;
wherein the point light source (67) has an emission angle of no less than 120°; and the lower brightness enhancement film (65) and the upper brightness enhancement film (66) each are provided with a prism with an adjustable installation angle;
wherein the light emission module (6) has a total thickness of no more than 0.95 mm;
wherein the light guide plate (63) has a uniformity of no less than 90%.

2. The intelligent photo-biological treatment device for eyes according to claim 1, wherein the device body (1) is further provided therein with a music playback module; and the music playback module is electrically connected with the controller.

3. The intelligent photo-biological treatment device for eyes according to claim 1, wherein the controller comprises a light emission control circuit; the light emission control circuit comprises an SY7201ABC microprocessor chip U4; a pin LX of the microprocessor chip U4 is connected with one terminal of an inductor L1 and a positive terminal of the infrared LED point light source; a negative terminal of the infrared LED point light source is connected with a first capacitor C3 and then grounded; a ground pin GND of the microprocessor chip U4 is grounded; a pin FB of the microprocessor chip U4 is connected with one terminal of a first resistor R13, one terminal of a second resistor R12, and a positive pole of a power supply; the other terminal of the first resistor R13 and the other terminal of the second resistor are grounded; a pin IN of the microprocessor chip U4 is connected with the other terminal of the inductor L1, one terminal of a second capacitor C2, and a 3.3 V voltage; the other terminal of the second capacitor C2 is grounded; a pin OVP of the microprocessor chip U4 is connected in series with a third resistor R9 and then connected with one terminal of the first capacitor C3 and a negative pole of the power supply; and pins EN/PWM of the microprocessor chip U4 are connected in series with a fourth resistor R10 and then connected with a power supply voltage VCC.

## Patentansprüche

1. Intelligente photobiologische Behandlungsvorrichtung für Augen, **dadurch gekennzeichnet, dass** sie einen Vorrichtungskörper (1) umfasst, wobei der Vorrichtungskörper (1) eine erste Abdeckung (2) und eine zweite Abdeckung (3) umfasst, die jeweils an das linke und das rechte menschliche Auge angepasst sind; wobei eine dem menschlichen Auge zugewandte Seite sowohl der ersten Abdeckung (2) als auch der zweiten Abdeckung (3) der Reihe nach mit einem ersten Hohlraum (4) und einem zweiten Hohlraum (5) versehen ist; wobei in dem ersten Hohlraum (4) ein Lichtemissionsmodul (6) vorgesehen ist; und wobei in dem zweiten Hohlraum (5) ein photobiologischer Trainingsraum vorgesehen ist, der in engem Kontakt mit dem menschlichen Auge steht;
wobei das Lichtemissionsmodul (6) ein Gummi-Eisen-Element (61) umfasst; wobei das Gummi-Eisen-Element (61) eine Montagenut umfasst; wobei in der Montagenut von unten nach oben der Reihe nach eine Reflexionsfolie (62), eine Lichtleiterplatte (63), eine Diffusionsfolie (64), eine untere Helligkeitsverstärkungsfolie (65), eine obere Helligkeitsverstärkungsfolie (66) und ein schwarz-schwarzes doppelseitiges Klebeband (68) vorgesehen sind; wobei das schwarz-schwarze doppelseitige Klebeband (68) an eine Innenseite der ersten Abdeckung (2) oder der zweiten Abdeckung (3) geklebt ist; wobei eine Punktlichtquelle (67) an einer Seite der Lichtleiterplatte (63) und zwischen der Reflexionsfolie und der Diffusionsfolie (64) angeordnet ist; und wobei die Punktlichtquelle (67) dazu eingerichtet ist, Infrarotlicht mit einer Wellenlänge von 600-750 nm zu emittieren; und
wobei in dem Vorrichtungskörper (1) eine Steuerung vorgesehen ist und die Steuerung elektrisch mit der Punktlichtquelle (67) verbunden ist;
wobei die Punktlichtquelle (67) eine Substratschicht umfasst; wobei auf der Substratschicht eine optische Bragg-Reflexionsschicht vorgesehen ist; wobei auf der optischen Bragg-Reflexionsschicht eine Begrenzungsschicht aus n-Typ-Aluminium-Gallium-Indium-Phosphid (N-AlGaInP) mit einer Dicke von 1.800-2.200 nm vorgesehen ist; wobei auf der N-AlGaInP-Begrenzungsschicht eine aktive I-AlGaInP-Schicht mit einer Dicke von 0,1-0,3 µm vorgesehen ist; wobei auf der aktiven I-AlGaInP-Schicht eine P-AlGaInP-Begrenzungsschicht mit einer Dicke von 34.000-36.000 nm vorgesehen ist; und wobei auf der P-AlGaInP-Begrenzungsschicht eine Fensterschicht aus p-Typ-Galliumphosphid (P-GaP) mit einer Dicke von 47.000-49.000 nm vorgesehen ist; und
wobei die Substratschicht ein Galliumarsenid-Substrat (GaAs) mit einer Dicke von 220-320 nm umfasst; wobei auf einer Oberfläche des GaAs-Substrats eine Siliziumdioxid-Schutzschicht (SiO₂) mit einer Dicke von 200-220 nm vorgesehen ist; und wobei auf einer Oberfläche der SiO₂-Schutzschicht eine Indiumzinnoxid-Verstärkungsschicht (ITO) mit einer Dicke von 200-250 nm vorgesehen ist;
wobei die Lichtleiterplatte (63) durch Lasergravur aus Polymethylmethacrylat (PMMA) hergestellt ist;
wobei die Punktlichtquelle (67) einen Emissionswinkel von mindestens 120° aufweist; und wobei die untere Helligkeitsverstärkungsfolie (65) und die obere Helligkeitsverstärkungsfolie (66) jeweils mit einem Prisma mit einstellbarem Einbauwinkel versehen sind;
wobei das Lichtemissionsmodul (6) eine Gesamtdicke von höchstens 0,95 mm aufweist;
wobei die Lichtleiterplatte (63) eine Gleichmäßigkeit von mindestens 90 % aufweist.

2. Intelligente photobiologische Behandlungsvorrichtung für Augen nach Anspruch 1, wobei in dem Vorrichtungskörper (1) ferner ein Musikwiedergabemodul vorgesehen ist; und wobei das Musikwiedergabemodul elektrisch mit der Steuerung verbunden ist.

3. Intelligente photobiologische Behandlungsvorrichtung für Augen nach Anspruch 1, wobei die Steuerung eine Lichtemissionssteuerschaltung umfasst; wobei die Lichtemissionssteuerschaltung einen SY7201ABC-Mikroprozessorchip U4 umfasst; wobei ein Pin LX des Mikroprozessorchips U4 mit einem Anschluss einer Induktivität L1 und einem positiven Anschluss der Infrarot-LED-Punktlichtquelle verbunden ist; wobei ein negativer Anschluss der Infrarot-LED-Punktlichtquelle mit einem ersten Kondensator C3 verbunden und anschließend geerdet ist; wobei ein Massepin GND des Mikroprozessorchips U4 geerdet ist; wobei ein Pin FB des Mikroprozessorchips U4 mit einem Anschluss eines ersten Widerstands R13, einem Anschluss eines zweiten Widerstands R12 und einem Pluspol einer Stromversorgung verbunden ist; wobei der andere Anschluss des ersten Widerstands R13 und der andere Anschluss des zweiten Widerstands geerdet sind; wobei ein Pin IN des Mikroprozessorchips U4 mit dem anderen Anschluss der Induktivität L1, einem Anschluss eines zweiten Kondensators C2 und einer Spannung von 3,3 V verbunden ist; wobei der andere Anschluss des zweiten Kondensators C2 geerdet ist; wobei ein Pin OVP des Mikroprozessorchips U4 mit einem dritten Widerstand R9 in Reihe geschaltet und anschließend mit einem Anschluss des ersten Kondensators C3 und einem Minuspol der Stromversorgung verbunden ist; und wobei Pins EN/PWM des Mikroprozessorchips U4 mit einem vierten Widerstand R10 in Reihe geschaltet und anschließend mit einer Versorgungsspannung VCC verbunden sind.

## Revendications

1. Dispositif intelligent de traitement photobiologique pour les yeux, **caractérisé en ce qu'**il comprend un corps de dispositif (1), dans lequel le corps de dispositif (1) comprend un premier cache (2) et un second cache (3) qui sont respectivement adaptés à l'œil gauche et à l'œil droit d'un être humain ; un côté de chacun du premier cache (2) et du second cache (3), orienté vers l'œil humain, est pourvu successivement d'une première cavité (4) et d'une seconde cavité (5) ; la première cavité (4) est pourvue intérieurement d'un module d'émission de lumière (6) ; et la seconde cavité (5) est pourvue intérieurement d'une chambre d'entraînement photobiologique en contact étroit avec l'œil humain ;
le module d'émission de lumière (6) comprend un élément en caoutchouc-fer (61) ; l'élément en caoutchouc-fer (61) comprend une rainure d'assemblage ; la rainure d'assemblage est pourvue intérieurement, successivement de bas en haut, d'un film réfléchissant (62), d'une plaque de guidage de lumière (63), d'un film diffusant (64), d'un film inférieur d'amélioration de la luminosité (65), d'un film supérieur d'amélioration de la luminosité (66) et d'un ruban adhésif double face noir-noir (68) ; le ruban adhésif double face noir-noir (68) est collé sur un côté intérieur du premier cache (2) ou du second cache (3) ; une source lumineuse ponctuelle (67) est située sur un côté de la plaque de guidage de lumière (63) et entre le film réfléchissant et le film diffusant (64) ; et la source lumineuse ponctuelle (67) est configurée pour émettre une lumière infrarouge de 600 à 750 nm ; et
le corps de dispositif (1) est pourvu intérieurement d'un contrôleur, et le contrôleur est connecté électriquement à la source lumineuse ponctuelle (67) ;
dans lequel la source lumineuse ponctuelle (67) comprend une couche de substrat ; la couche de substrat est pourvue d'une couche de réflexion optique de Bragg ; la couche de réflexion optique de Bragg est pourvue d'une couche de confinement en phosphure d'aluminium-gallium-indium de type N (N-AlGaInP), ayant une épaisseur de 1 800 à 2 200 nm ; la couche de confinement en N-AlGaInP est pourvue d'une couche active en I-AlGaInP, ayant une épaisseur de 0,1 à 0,3 µm ; la couche active en I-AlGaInP est pourvue d'une couche de confinement en P-AlGaInP, ayant une épaisseur de 34 000 à 36 000 nm ; et la couche de confinement en P-AlGaInP est pourvue d'une couche-fenêtre en phosphure de gallium de type p (P-GaP), ayant une épaisseur de 47 000 à 49 000 nm ; et
la couche de substrat comprend un substrat en arséniure de gallium (GaAs), ayant une épaisseur de 220 à 320 nm ; une surface du substrat en GaAs est pourvue d'une couche protectrice en dioxyde de silicium (SiO₂), ayant une épaisseur de 200 à 220 nm ; et une surface de la couche protectrice en SiO₂ est pourvue d'une couche de renforcement en oxyde d'indium-étain (ITO), ayant une épaisseur de 200 à 250 nm ;
dans lequel la plaque de guidage de lumière (63) est fabriquée en polyméthacrylate de méthyle (PMMA) par gravure laser ;
dans lequel la source lumineuse ponctuelle (67) présente un angle d'émission qui n'est pas inférieur à 120° ; et le film inférieur d'amélioration de la luminosité (65) et le film supérieur d'amélioration de la luminosité (66) sont chacun pourvus d'un prisme dont l'angle d'installation est réglable ;
dans lequel le module d'émission de lumière (6) présente une épaisseur totale qui n'est pas supérieure à 0,95 mm ;
dans lequel la plaque de guidage de lumière (63) présente une uniformité qui n'est pas inférieure à 90 %.

2. Dispositif intelligent de traitement photobiologique pour les yeux selon la revendication 1, dans lequel le corps de dispositif (1) est en outre pourvu intérieurement d'un module de lecture musicale ; et le module de lecture musicale est connecté électriquement au contrôleur.

3. Dispositif intelligent de traitement photobiologique pour les yeux selon la revendication 1, dans lequel le contrôleur comprend un circuit de commande d'émission de lumière ; le circuit de commande d'émission de lumière comprend une puce de microprocesseur SY7201ABC U4 ; une broche LX de la puce de microprocesseur U4 est connectée à une borne d'une inductance L1 et à une borne positive de la source lumineuse ponctuelle à DEL infrarouge ; une borne négative de la source lumineuse ponctuelle à DEL infrarouge est connectée à un premier condensateur C3, puis mise à la terre ; une broche de masse GND de la puce de microprocesseur U4 est mise à la terre ; une broche FB de la puce de microprocesseur U4 est connectée à une borne d'une première résistance R13, à une borne d'une deuxième résistance R12 et à un pôle positif d'une alimentation électrique ; l'autre borne de la première résistance R13 et l'autre borne de la deuxième résistance sont mises à la terre ; une broche IN de la puce de microprocesseur U4 est connectée à l'autre borne de l'inductance L1, à une borne d'un deuxième condensateur C2 et à une tension de 3,3 V ; l'autre borne du deuxième condensateur C2 est mise à la terre ; une broche OVP de la puce de microprocesseur U4 est connectée en série à une troisième résistance R9, puis connectée à une borne du premier condensateur C3 et à un pôle négatif de l'alimentation électrique ; et des broches EN/PWM de la puce de microprocesseur U4 sont connectées en série à une quatrième résistance R10, puis connectées à une tension d'alimentation VCC.
